# EUROPÄISCHE PATENTSCHRIFT

(11) **EP 1 471 093 B1**
(45) Veröffentlichungstag und Bekanntmachung des Hinweises auf die Patenterteilung: **18.04.2007**
(21) Anmeldenummer: 04008705.8
(22) Anmeldetag: 13.04.2004
(51) Int. Cl.: C08J 3/00, B29C 47/46

(54) **Verfahren und Vorrichtung zur Herstellung von elastomermodifizierten Thermoplasten**
Process and apparatus for producing thermoplastic resins modified with an elastomer
Procédé et appareil pour fabriquer une résine thermoplastique modifiée à l'aide d'un élastomère

(30) Priorität: 22.04.2003 DE 10318109
(43) Veröffentlichungstag der Anmeldung: 27.10.2004
(73) Patentinhaber: LANXESS Deutschland GmbH, 51369 Leverkusen (DE)
(72) Erfinder: Eitel, Alfred, Dr., 8222 St. Johann (AT); Jonsson, Haakan, Dr., Cranberry Twp, PA 16066 (US); Liesenfelder, Ulrich, 51469 Bergisch Gladbach (DE); Moss, Stefan, Dr., 42781 Haan (DE); Nothelle, Ricarda, Dr., 51371 Leverkusen (DE); Frankenau, Andreas, Dr., 47906 Kempen (DE); Böhmer, Stephen, 50259 Pulheim (DE)

(56) Entgegenhaltungen:
- EP-A- 0 735 077
- EP-A- 0 867 463
- WO-A-02/28931
- GB-A- 1 399 504

## Beschreibung

Die vorliegende Erfindung betrifft elastomermodifizierte Thermoplaste und thermoplastische Formmassen mit niedrigen Monomerrestgehalten und heller Farbe und ein Verfahren sowie eine Vorrichtung zu ihrer Herstellung.

Polymere weisen stets einen Restanteil von Monomeren, aus welchen sie aufgebaut sind, und eine Eigenfarbe auf. Häufig ist die Entfernung der Restmonomeren aus den Formmassen mit einer thermischen Belastung verbunden, was zu einer unerwünschten Intensivierung der Eigenfarbe führt. Zur Verbesserung der Eigenschaften ist es wesentlich, die Restmonomeren in geeigneter Weise zu entfernen.

In der Praxis ist generell eine helle Eigenfarbe erwünscht. Bei eingefärbten Produkten erzeugt dies auch einen wirtschaftlichen Nutzen, weil die Menge an Farbstoff zur Einfärbung reduziert werden kann und nicht in verstärktem Maß zur Überdeckung einer gelblichen Eigenfarbe eingesetzt werden muss.

Eine Entfernung von Restmonomeren auf chemischem Weg wird beispielsweise in der EP-A1-0 768 337 beschrieben. Die Entfernung der Restmonomeren erfolgt dabei durch Zusatz CH-acider organischer Verbindungen. Durch dieses bekannte Verfahren lassen sich Monomere wie Styrol und Acrylnitril zuverlässig entfernen, jedoch keine Lösemittel oder Nebenprodukte der Polymerisation. Der gleiche Mangel tritt beim Verfahren zur Reduzierung der Restmonomeren mit Alkali-(bi)-sulfid auf (DE-A1-2 546 182). Die chemische Umwandlung von Restmonomeren führt jedoch unter Umständen zu Produkten mit unerwünschter ökologischer Relevanz, wodurch der Einsatz dieser Produkte in der Praxis deutlich erschwert wird.

Bei einem anderen bekannten Verfahren werden die Restmonomere in einem zusätzlichen Verfahrensschritt in Extrudern mit und ohne Wasserzusatz unter Vakuum entfernt. Der zusätzliche Verfahrensschritt führt zu einer Kostenerhöhung und einer weiteren unerwünschten Farbvertiefung der Produkte.

Als aufwendig erweist sich ein Verfahren zur Entfernung von Restmonomeren durch Injektion von superkritischen Lösungsmitteln in die Polymerschmelze (EP-A1-0 798 314).

Ein weiteres Verfahren zur Herstellung elastomermodifizierter Thermoplaste ist in der EP-A1-0 867 463 beschrieben. In einem Mischkneter wird feuchtes, restmonomerenhaltiges mit Styrol und Acrylnitril gepfropftes Polybutadienpulver mit Poly-Styrolacrylnitrilschmelze vermengt und unter gleichzeitiger Verdampfung des Wassers von Restmonomeren befreit. Das fertige ABS wird am Ende des Ausdampfapparats ausgetragen und in Granulatform überführt. Nachteilig an diesem Verfahren ist, dass die erzielbare Durchsatzleistung begrenzt ist. Bei hoher Durchsatzleistung wird Pulver in erheblichem Maß nicht in die Schmelze eingearbeitet, sondern mit den Brüden mitgerissen. Dies führt zu Ausbeuteverlusten und zu instabilen Betriebszuständen. Um dem entgegenzuwirken, kann durch die Betriebsweise des Mischkneters ein Rückstau an Schmelze am Austrag des Mischkneters erzeugt werden. Diese Schmelze soll das Pulver binden und am Austreten hindern. Diese Verfahrensweise ist jedoch nur in eingeschränktem Maße nutzbar.

Ausgehend von diesem bekannten Verfahren, liegt der vorliegenden Erfindung die Aufgabe zugrunde, ein Verfahren Verfügung zu stellen, das die geschilderten Nachteile nicht aufweist.

Gegenstand der Erfindung ist ein Verfahren zur Herstellung von elastomermodifizierten Thermoplasten durch Mischen von Elastomer D), welches durch Pfropfpolymerisation mit einer Hülle aus einem thermoplastischen Kunststoff A) umgeben ist, mit einem thermoplastischen Kunststoff B),
wobei das feuchte gepfropfte Elastomer D) auf 1 bis 50 Gew.-%, insbesondere 5 bis 50 Gew.-%, insbesondere bevorzugt 10 bis 40 Gew.-%, Restfeuchte vorentwässert und in den als Schmelze vorliegenden thermoplastischen Kunststoff B) eingemischt wird, wobei das Schmelzen des Elastomeren D), das Legieren des Elastomeren D) mit der Schmelze des thermoplastischen Kunststoffs B), sowie die Entfernung von weiteren organischen flüchtigen Bestandteilen C) in einem kontinuierlich durchlaufenen Knetreaktor mit Knetbarren erfolgt, der so aufgebaut ist, dass ein Teil der Knetbarren das Kunststoffmaterial in Richtung des Reaktoreinlasses rückfördert.

Bevorzugt ist eine Ausführungsform des Verfahrens, bei der die zum Aufschmelzen, Aufheizen und Ausdampfen der Polymermischung notwendige Energie mechanisch über die Knetwirkung der Rotoren und thermisch über die Gehäuseflächen eines Knetreaktors eingebracht wird, wobei das Verhältnis zwischen in die Mischung einzubringender mechanischer und thermischer Energie von 4 : 1 bis 1 : 6, insbesondere von 2,5 : 1 bis 1 : 4, beträgt.

Bevorzugt wird der Prozess in einem großvolumigen, teilweise gefüllten Knetreaktor mit rotierenden Einbauten durchgeführt, in dem pro Liter Prozessraum nicht mehr als 5 kg/h Polymer durchgesetzt werden.

Besonders bevorzugt ist ein Verfahren, dadurch gekennzeichnet, dass bei der Mischung von gepfropftem Elastomer D) und Thermoplast B) zusätzlich Farb- und/oder Zuschlagstoffe zugegeben werden.

Besonders bevorzugt werden Farb- und/oder Zuschlagstoffe der Polymermischung in einem dem Knetreaktor nachgeschalteten statischen Mischer zugegeben.

Nach einer Variante wird das Verfahren bevorzugt in einem Knetreaktor mit Knetreaktoreinbauten durchgeführt, deren spezifische Antriebsleistung von 0,01 bis 1 kWh pro kg trockene Polymerschmelze, bevorzugt 0,05 bis 0,5 kWh/kg und besonders bevorzugt 0,05 bis 0,25 kWh/kg, beträgt.

Bevorzugt ist ein Verfahren, dadurch gekennzeichnet, dass das Vorentwässern des Elastomeren D) in einer Zentrifuge, einer Entwässerungsschnecke oder in einer Sequenz beider Aggregate erfolgt.

Die Vorentwässerung erfolgt besonders bevorzugt in einer kontinuierlichen Abpressvorrichtung, gegebenenfalls mit variabler Stauvorrichtung, insbesondere in einer Schneckenpresse.

Insbesondere wird die Vorentwässerung bei einer Temperatur unterhalb der Glastemperatur des Thermoplasten A), bevorzugt kleiner 90°C, ausgeführt.

Bei der Vorentwässerung wird besonders bevorzugt eine Energie von 0,016 bis 0,05 kWh/kg Polymer, bevorzugt 0,018 bis 0,03 kWh/kg Polymer, aufgewendet.

Der Wassergehalt des Produktes der Vorentwässerung beträgt 1 bis 50 Gew.-%, bevorzugt 5 bis 50 Gew.-%, besonders bevorzugt 10 bis 40 Gew.-%. Wenn das Vorentwässern des Elastomeren D) in einer Zentrifuge und einer nachgeschalteten Entwässerungsschnecke erfolgt, beträgt die Restfeuchte des Elastomeren D) nach der Vorentwässerung insbesondere 10 bis 23 Gew.-%, besonders bevorzugt 16 bis 21 Gew.-%.

Aus der EP-A1-0 734 825 ist ein Verfahren zur Herstellung von Thermoplasten bekannt, bei welchem Wasser aus einer Elastomerkomponente in einem Extruder ZSK der Fa. Werner und Pfleiderer abgepresst und ausdrücklich die Verwendung einer Seiherschnecke als störanfällig beschrieben wird. Dort wird auch darauf hingewiesen, dass die Ausdampfung von viel Wasser aufwendig ist.

Dem Vorurteil aus dem gattungsbildenden Stand der Technik entgegen, gelingt es bei der vorliegenden Erfindung, Wasser mit einer Seiherschnecke vorteilhaft abzupressen und den Vorteil der Reinigungswirkung mittels einer Wasserdampfdestillation in einem geeigneten Folgeapparat nach der Vereinigung der Elastomerkomponente mit der kautschukfreien Komponente zu nutzen. Die Verwendung von zwei Apparaten erhöht die Flexibilität des erfindungsgemäßen Verfahrens.

Als Elastomer D) kann im erfindungsgemäßen Verfahren jedes Polymer eingesetzt werden, das elastomere Eigenschaften hat und einem Extruder zugeführt werden kann. Bevorzugt werden als Elastomere D) Kautschuke eingesetzt. Die Elastomere D), insbesondere Kautschuke, sind mit einem thermoplastischen Kunststoff A) gepfropft. Geeignete Kautschuke sind z.B. Nitrilkautschuke bzw. teilverseifte Nitrilkautschuke. Insbesondere werden teilchenförmige Kautschuke verwendet.

Bevorzugte Pfropfpolymerisate D) sind z.B. mit Styrol gepfropfte Polybutadiene, Butadien/Styrol-Copolymerisate, d.h. Copolymerisate der in der DE-A1-1 694 173 beschriebenen Art; Acrylnitril, Styrol und/oder Alkylstyrolen gepfropfte Polybutadiene, Butadien/Styrol- oder Butadien/Acrylnitril-Copolymerisate, Polyisobutene oder Polyisoprene, wie sie beispielsweise in der DE-A1-2 348 377 beschrieben sind.

Besonders bevorzugte Pfropfpolymerisate D) sind ABS-Polymerisate, wie sie beispielsweise in der DE-A1-2 035 390 oder DE-A1-2 248 242 beschrieben sind.

Zur Durchführung des erfindungsgemäßen Verfahrens geeignete Elastomere D) (Pfropfpolymere) sind pfropfpolymerisierte Vinylverbindungen wie z.B. von Styrol, α-Methylstyrol, Acrylnitril, Methylmethacrylat und Alkylacrylat oder deren Mischungen; besonders bevorzugt sind Methylmethacrylat sowie Mischungen von Styrol und Acrylnitril, von α-Methylstyrol und Acrylnitril, von Methylmethacrylat und Styrol, von Methylmethacrylat und Alkylacrylat, von α-Methylstyrol, Methylmethacrylat und Acrylnitril. Als Kautschuke, die als bevorzugte Pfropfgrundlage dienen, kommen Dienhomo- und -Copolymerisate z.B. von Butadien, Isopren, Chloropren, gegebenenfalls mit bis zu 35 Gew.-% Comonomeren wie Styrol, Acrylnitril, Methylmethacrylat, Alkylacrylat, Vinylmethylether in Betracht oder Alkylacrylat-Polymerisate (insbesondere von C₁-C₁₀-Alkylacrylaten), die gegebenenfalls bis zu 20 Gew.-% Vinylmonomere, wie Styrol, Acrylnitril, Vinylacetat, C₁-C₁₀-Alkylmethacrylat einpolymerisiert enthalten; die Acrylatkautschuke sind gegebenenfalls durch Einpolymerisieren polyfunktioneller Vinyl- oder Allylmonomere partiell vernetzt; vernetzende Monomere sind z.B. Bis-Acrylate, Bis-Acrylamide, Butadien, Acrylsäurevinylester, Triallylcyanurat, Trialkylisocyanurat, Zitronensäuretrisallylester, Bis-Carbonsäurevinylester.

Weitere Beispiele für geeignete Kautschuke sind Acrylatkautschuke. Acrylatkautschuke enthalten die vernetzenden Monomere in Mengen bis maximal 5 Gew.-%. Die Kautschuke können auch Kern/Mantel-Struktur besitzen, d.h. die Acrylatkautschukpartikel enthalten einen Kautschukkern, der sich strukturell von dem ihn umgebenden Acrylatkautschuk unterscheidet, oder einen harten Thermoplastharzkern. Insbesondere können Stufen aus einem oder mehreren der Monomeren Styrol, Alkylstyrol, Acrylnitril, Methylmethacrylat polymerisiert werden. Bevorzugt sind Pfropfpolymeren auf Basis von Butadien/Styrol/Acrylnitril, n-Butylacrylat/Styrol/Acrylnitril, Butadien/n-Butylacrylat/Styrol/Acrylnitril, n-Butylacrylat/Styrol/Methylmethacrylat, Butadien/Styrol/Acrylnitril/Methylmethacrylat und Butadien/n-Butylacrylat/Methylmethacrylat/Styrol/-Acrylnitril.

Erfindungsgemäß werden mit den Pfropfpolymeren (Elastomere D) zur Herstellung von elastomermodifiziererten Thermoplasten thermoplastische Kunststoffe B), bevorzugt Styrol-Acrylnitril-(SAN)-Copolymere, Polystyrol, Polymethylmethacrylat, Polyvinylchlorid oder Mischungen dieser Polymere, gemischt.

Dabei sind SAN-Polymere, Polymethylmethacrylat (PMMA) oder Mischungen dieser Polymere besonders bevorzugt als thermoplastische Kunststoffe B). Besonders bevorzugt eingesetzte Copolymerisate werden erhalten aus 2 bis 50 Gew.-%, insbesondere 20 bis 40 Gew.-%, Acrylnitril und 50 bis 98 Gew.-%, insbesondere 60 bis 80 Gew.-%, Styrol, α-Methylstyrol, kernsubstituiertem Styrol oder Mischungen aus diesen. Sie sind bekannt und lassen sich durch radikalische Polymerisation, z.B. in Emulsion, Suspension, Lösungs- oder Masse, herstellen. Die Copolymerisate besitzen vorzugsweise ein Molekulargewicht M_{w} von 15 000 bis 200 000 (Gewichtsmittel, ermittelt durch Lichtstreuung oder Sedimentation).

Weiterhin können als thermoplastische Kunststoffe B) auch Polycarbonat, Polybutylenterephthalat, Polyoxymethylen, Polymethylmethacrylat, Polyphenylensulfid, Polysulfone, Polyethersulfone und Polyamide und Mischungen dieser Thermoplasten eingesetzt werden.

Die Pfropfpolymerisate D) können nach bekannten Verfahren, wie Suspensions- oder Emulsionsverfahren, hergestellt werden. Die Vinylpolymerisatlatices können z.B. in bekannter Weise durch Emulsionspolymerisation in wässrigen Medien bzw. Emulsionspfropfpolymerisation in Gegenwart von Kautschuklatices hergestellt werden. Im Falle von kautschukfreien Polymerisaten werden die Monomere bei pH-Werten von ca. 12 bis 2, insbesondere von 10 bis 3, radikalisch in Gegenwart von Seifen (Emulgatoren) in wässrigem Medium polymerisiert. Als Initiatoren kommen insbesondere wasserlösliche Radikalbildner wie Peroxodisulfate, Peroxodiphosphate, wasserlösliche Hydroperoxide und Peroxosäuren in Betracht, sowie Redoxinitiatorsysteme. Die Polymerisation, die normalerweise bei 40 bis 90°C durchgeführt wird, erfordert die Anwesenheit eines ionischen Emulgators, insbesondere eines anionischen Emulgators, in Mengen bis zu 4 Gew.-%, bevorzugt bis zu 2,5 Gew.-%, bezogen auf die Monomere. Geeignete Emulgatoren sind beispielsweise Fettsäuresalze, Alkylsulfonsäuresalze mit längerkettigen Alkylresten und Schwefelsäurealkylhalbester mit längerkettigen Alkylresten sowie bevorzugt Alkalisalze der disproportionierten Abietinsäure.

Bei der Pfropfpolymerisation werden die Pfropfmonomeren bekanntlich nicht vollständig auf die Pfropfgrundlage polymerisiert; erfindungsgemäß schließen Pfropfpolymerisate D) aber Produkte ein, die durch Polymerisation der Pfropfmonomere in Gegenwart der Pfropfgrundlage gewonnen werden.

Die so hergestellten Vinylpolymerisatlatices weisen i.a. einen Polymerfeststoffgehalt von 10 bis 70 Gew.-%, vorzugsweise von 25 bis 50 Gew.-% auf. Der Anteil an nicht polymerisierten Monomeren im Latex beträgt in der Regel 0 bis 15 Gew.-%, vorzugsweise 0 bis 5 Gew.-% bezogen auf den Polymerfeststoffgehalt des Latex. Die Größe der Vinylpolymerisat-Latexteilchen beträgt 50 bis 1 000 nm, vorzugsweise 80 bis 650 nm.

Besonders bevorzugt sind Pfropfkautschuke mit Kautschukgehalten von mindestens 50 Gew.%, vorzugsweise mindestens 55 Gew.-%.

Besonders bevorzugte Pfropfpolymerisate D) sind erhältlich durch Pfropfpolymerisation von 30 bis 90 Gew.-%, vorzugsweise 50 bis 85 Gew.-%, insbesondere 60 bis 80 Gew.-%, bezogen auf Pfropfpolymerisat D), eines Butadienpolymerisats mit mindestens 50 Gew.-% Butadienresten (als Pfropfgrundlage).

Das Butadienpolymerisat kann neben Butadienresten bis zu 50 Gew.-% Reste anderer ethylenisch ungesättigter Monomere, wie Styrol, Acrylnitril, enthalten. Bevorzugt ist Polybutadien.

Im Allgemeinen beträgt der Gelanteil der Pfropfgrundlage mindestens 20 Gew.-% (in Toluol gemessen), der Pfropfgrad G 0,15 bis 0,55. Der Gelgehalt der Pfropfgrundlage wird bei 25°C in Dimethylformamid bestimmt (M. Hoffmann, H. Krömer, R. Kuhn, Polymeranalytik I und II, Georg-Thieme-Verlag, Stuttgart 1977).

Die Latices werden nach grundsätzlich bekannten Verfahren koaguliert (siehe z.B. EP 459 161 A2).

Als Koagulans verwendet man vorzugsweise wässrige Lösungen von wasserlöslichen anorganischen oder organischen Säuren und/oder Salzen, wie Salzsäure, Schwefelsäure, Phosphorsäure, Borsäure, Ameisensäure, Essigsäure, Propionsäure, Zitronensäure, Alkali-, Erdalkali-, Zink- und Aluminium-chloride-, -sulfate, -formiate, -acetate, -phosphate, -carbonate, Aluminate, teilverseifte Polyvinylacetate gegebenenfalls in Kombinationen mit anorganischen oder organischen Säuren. Je nach zu koagulierender Vinylpolymerisatlatexmischung benutzt man 0,2 bis 25 gew.-%ige wässrige Lösungen.

Unter organischen flüchtigen Bestandteilen C) werden Monomere und niedermolekulare Oligomere der eingesetzten thermoplastischen Polymere bzw. der Elastomere oder Pfropfpolymere (z.B. Styrol, Acrylnitril), Emulgatorbestandteile (z.B. Dihydroabietinsäure) oder Nebenprodukte der Polymerisation sowie Lösemittel, die bei der Herstellung der Pfropfpolymere bzw. Thermoplasten eingesetzt werden (z.B. Ethylbenzol, MEK), verstanden.

Der für die Pfropfung des Elastomeren D) verwendete thermoplastische Kunststoff A) kann einer der thermoplastischen Kunststoffe B) oder eine Mischung daraus sein. Als thermoplastischer Kunststoff A) kann der gleiche oder ein anderer als der thermoplastische Kunststoff B) verwendet werden. Der thermoplastische Kunststoff A) ist bevorzugt ausgewählt aus der Reihe Styrol-Acrylnitril-(SAN)-Copolymere, Polystyrol, Polymethylmethacrylat, Polyvinylchlorid, Polycarbonat, Polybutylenterephthalat, Polyoxymethylen, Polymethylmethacrylat, Polyphenylensulfid, Polysulfone, Polyethersulfone und Polyamide und Mischungen dieser Thermoplasten.

Knetreaktoren im Sinne der Erfindung sind Reaktoren mit Knetwellen zum Eintrag mechanischer Energie in das zu behandelnde Mischgut, wobei die Knetwellen längs ihrer Achse mit einer Mehrzahl von, nicht notwendigerweise runden, Knetscheiben versehen sind. Die Scheiben sind mit mindestens einem axial ausgerichteten und den Umfang des Gehäuses abschabenden Werkzeug, den sogenannten "Knetbarren" versehen. Üblicherweise gibt man den Knetbarren einen Anstellwinkel zur Längsachse der Maschine, so dass sie einen Transport des zu verarbeitenden Produktes vom Eintritt zum Austritt bewerkstelligen. Der Knetreaktor weist mindestens zwei Eingänge für das Misch-/Reaktionsgut und in seinem Endbereich einen Austrag für das Polymere sowie einen Auslass für die freiwerdenden Dämpfe auf. Eine vorteilhafte Anordnung der Ein- und Auslässe wird in EP-A1-0 867 463 beschrieben.

Erfindungsgemäß wird ein Knetreaktor eingesetzt, bei dem ein Teil der Knetbarren das Kunststoffmaterial in Richtung des Reaktoreinlasses rückfördert. In einer besonders bevorzugten Variante des Verfahrens erfolgt die Rückförderung der Knetbarren im Auslassbereich des Knetreaktors.

Überraschend wurde gefunden, dass ohne die oben beschriebenen Nachteile helle Produkte mit niedrigen Restmonomeren zur Verfügung gestellt werden können, wenn bei der Vereinigung von Schmelze z.B. der Produkte B) und feuchten Pulvern der Produkte D) in einem Mischkneter an den Knetwellen rückfördernde Knetbarren angebracht werden, wie weiter unten noch näher beschrieben wird. Die rückfördernden Knetbarren werden bevorzugt nahe dem Produktaustrag angebracht, besonders bevorzugt auf 15 bis 25 % der produktberührten Länge der Kneterachse vom Produktaustrag an. Die erfindungsgemäß angebrachten Knetbarren wirken durch diese Anstellung dem Transport des Produktes in der Maschine entgegen.

Überraschenderweise wurde gefunden, dass trotz verschlechterter Transportwirkung der Durchsatz bedingt durch diese Maßnahme erheblich gesteigert werden kann. Eine erhöhte Leistungsaufnahme des Mischkneterantriebs erlaubt es, eine höhere Menge an Wasser zu verdampfen.

Ein weiterer Vorteil der höheren Knetleistung besteht darin, dass die Temperatur, mit der die Apparatewände beheizt werden, gesenkt werden kann. Die Beheizung erfolgt mit einer Temperatur von 200 bis 230°C, bevorzugt 200 bis 220°C. Die an den Wandungen anhaftenden Schichten werden dadurch thermisch geringer belastet. Weiterhin ist es möglich, im Vergleich zu der in der EP-A1-0 867 463 beschriebenen Verfahrensweise, niedrigere Drehzahlen einzusetzen, wodurch die Scherbelastung in den Spalten zwischen den Knetbarren und der Gehäusewand und zwischen den Knetbarren und dem gegenüberliegenden Rotor nochmals deutlich reduziert werden können. Durch die rückfördernd angestellten Knetbarren wird die Benetzung der stromaufwärts liegenden Einbauten gefördert, so dass das Pulver gebunden und nicht vom vorbeiströmenden Wasserdampf mitgerissen wird. Die Reinigung von den Restflüchtigen erfolgt schonend im Wasserdampfstrom.

Überraschend wurde auch gefunden, dass zusätzlich eine schonende Vorbehandlung von feuchten Pulvern (Elastomere D) in einer kontinuierlichen Abpressvorrichtung bei tiefer Temperatur derartig durchgeführt werden kann, dass keine oder nur eine minimale Plastifizierung erfolgt, so dass Qualitätseinbußen vermieden werden können. Die kontinuierliche Entwässerung erfolgt durch einen an sich bekannten Schneckenförderer, dessen Gehäuse zur Abfuhr des Wassers mit Schlitzen versehen ist. Zu diesem Zweck kann das Gehäuse aus zusammengehaltenen Stäben aufgebaut sein oder Siebflächen aufweisen. Da das Polymer in den Entwässerungszonen nicht plastifiziert, kommt es nicht zum Verstopfen der Entwässerungsschlitze.

Weiterer Gegenstand der Erfindung ist ein Knetreaktor, insbesondere zur Durchführung eines Verfahrens nach Anspruch 1, mindestens ausgestattet mit zwei achsparallelen Wellen, wobei auf beiden Wellen Scheiben mit an ihrem Umfang angeordneten Knetbarren zur Förderung des Reaktionsgemisches vorgesehen sind, mit einem die Einbauten umschließenden Gehäuse mit mind. zwei Einlässen und einem Austrag für das Produkt, einem Antriebsmittel für die gleich- oder gegensinnige Rotation der Wellen sowie einer Heiz-/Kühleinrichtung, dadurch gekennzeichnet, dass ein Teil der Knetbarren einen solchen Anstellwinkel zu ihrer Bewegungsrichtung aufweisen, dass sie bei einer durch das Antriebsmittel bewirkten Drehung der Wellen das Produkt vom Austrag weg in Richtung der Einlässe fördern.

Besonders bevorzugt ist ein Knetreaktor, dadurch gekennzeichnet, dass die an den drei bis fünf zum Austrag nächststehend angeordneten Scheiben befindlichen Knetbarren einen Anstellwinkel mit Rückförderungswirkung aufweisen.

Ein Knetbarren ist dann rückfördernd, wenn der Normalenvektor auf seiner Schubflanke eine Komponente entgegen der Stoffflussrichtung hat. Die Schubflanke ist diejenige Seite des Knetbarrens, die durch die Drehbewegung, welche der Knetbarren ausführt, angeströmt wird.

Weiterer Gegenstand der Erfindung sind Formmassen hergestellt aus elastomermodifizierten Thermoplasten erhalten nach einem oben genannten Verfahren sowie Formkörper hergestellt aus diesen Formmassen.

Die Schneckenwelle ist entweder durchgehend mit förderaktivem Schneckengewinde versehen oder mit einem an einer oder mehreren Stelle/n unterbrochenen Gewinde. In den Unterbrechungen des Gewindes werden dann vorteilhaft am Gehäuse Bolzen angebracht, die eine Rotation des Pulvers mit der Schnecke verhindern. Um eine noch bessere Abpresswirkung zu erzielen, wird der freie Querschnitt am Austritt des Schneckenförderers teilweise verschlossen. Bevorzugt wird der freie Querschnitt um 50 bis 95 % reduziert. Besonders bevorzugt geschieht dies durch eine variable Drosseleinrichtung in Form von beweglichen Schiebern oder Lochblenden. Am Austritt der Schnecke kann zur Steigerung der Stauwirkung ein konisch erweiterter Schneckenkemdurchmesser eingesetzt werden. Vorzugsweise werden außerhalb der Schneckenförderzonen konisch zulaufende oder sich verengende Kanäle vermieden. Auf diese Weise werden 60 bis 85 % des anhaftenden Wassers entfernt, wobei lediglich 0,016 bis 0,05 kWh/kg, bevorzugt 0,018 bis 0,03 kWh/kg, an Energie pro kg Polymer aufgewendet werden müssen. Das erfindungsgemäße Verfahren ist somit sehr energieeffizient. Ausgehend von einer Anfangsfeuchte von 25 bis 60 % werden 60 bis 85 % des im Feuchtgut enthaltenen Wassers entfernt. Man erzielt ohne Plastifizierung oder mit einem sehr geringen plastifizierten Anteil im Pulver (Elastomere D) Wassergehalte von 10 bis 23 %.

Die erfindungsgemäße Abpressung führt ebenfalls zu einer Minderung des Pulverflugs und ermöglicht eine erhebliche Steigerung des Durchsatzes. Sie trägt dazu bei, dass das Verfahren betriebssicher ist und restmonomerearme und farbhelle Formmassen hergestellt werden können.

Ein weiterer Vorteil der erfindungsgemäßen Abpressweise ist eine Reduzierung des Gehaltes an Salzen, welche aus der Polymerisation stammen und üblicherweise durch Wäschen entfernt werden müssen.

Erfindungsgemäß können die beschriebenen Homo- bzw. Copolymerisate teilweise durch andere Thermoplaste ersetzt werden. Die anderen Thermoplaste sind vorzugsweise ausgewählt aus mindestens einem Thermoplasten der Gruppe der Polycarbonate, Polyestercarbonate, Polyester, vorzugsweise Polyalkylenterephthalate.

Weiterhin können die erfindungsgemäßen Formmassen weitere Zusatzstoffe ausgewählt aus mindestens einem der Gruppe der Flammschutzmittel, Anti-Dripping-Mittel, feinstteilige anorganische Verbindungen und Füll- und Verstärkungsstoffe enthalten.

Mischung aus kautschukfreien Vinylpolymerisaten (thermoplastische Kunststoffe B) und kautschukhaltigen Vinylpolymerisaten (Elastomere D) enthalten vorzugsweise insbesondere
a. 0,5 bis 90 Gew.-Teile, vorzugsweise 10 bis 80 Gew.-Teile, insbesondere 25 bis 75 Gew.-Teile B) und
b. 10 bis 99,5 Gew.-Teile, vorzugsweise 20 bis 90 Gew.-Teile, insbesondere 75 bis 25 Gew.-Teile D) (bezogen auf B) und D)),
   wobei die Mischung wenig Restmonomere, nämlich kleiner/gleich 3ppm Acrylnitril und kleiner/gleich 20 ppm Vinylcyclohexen und 20 ppm Ethylbenzol und kleiner/gleich 80 ppm Styrol enthält und einen Yellowness-Index kleiner/gleich 30 aufweist.

Weiterhin sind besonders geeignete Mischungen auch solche, die B) und D) sowie andere Thermoplaste und ggf. weitere Zusatzstoffe enthalten. Andere Thermoplaste sind vorzugsweise ausgewählt aus der Gruppe der Polycarbonate, Polyestercarbonate, Polyalkylenterephtalate, Polyamide oder Mischungen daraus.

Die Zusammensetzungen enthalten vorzugsweise
b. 10 bis 70 Gew.-%, insbesondere 15 bis 60 Gew.-%, ganz besonders bevorzugt 20 bis 40 Gew.-% Polycarbonat, Polyestercarbonat, Polyalkylenterphthalat oder Gemische daraus oder Polyamid,
c. 90 bis 30 Gew.-%, vorzugsweise 85 bis 40 Gew.-%, insbesondere 80 bis 60 Gew.-% B) und D),
wobei die Summe der Gew.-% aller Komponenten 100 ergibt, mit der Maßgabe, dass mindestens eine der Komponenten B) und D) oder Mischungen daraus wenig Restmonomere, nämlich kleiner/gleich 3 ppm Acrylnitril und kleiner/gleich 20 ppm Vinylcyclohexen und 20 ppm Ethylbenzol und kleiner/gleich 80 ppm Styrol enthalten und einen Yellowness-Index kleiner/gleich 30 aufweisen.

Geeignete Polycarbonate und/oder Polyestercarbonate sind bekannt oder nach kannten Verfahren herstellbar (zur Herstellung aromatischer Polycarbonate siehe beispielsweise Schnell, "Chemistry and Physics of Polycarbonates", Interscience Publishers, 1964 sowie DE-A1-3 832 396; zur Herstellung aromatischer Polyestercarbonate beispielsweise die DE-A1-3 077 934).

Die erfindungsgemäßen thermoplastischen Formmassen eignen sich aufgrund ihrer sehr guten mechanischen Eigenschaften zur Herstellung von Formkörpern jeglicher Art, insbesondere solchen mit erhöhten Anforderungen an Bruchbeständigkeit. Insbesondere können Formkörper durch Spritzguss hergestellt werden. Beispiele für herstellbare Formkörper sind: Gehäuseteile jeder Art, z.B. für Haushaltsgeräte wie Saftpressen, Kaffeemaschinen, Mixer, für Büromaschinen wie Monitore, Drucker, Kopierer, oder Abdeckplatten für den Bausektor und Teile für den Kfz-Bereich. Sie sind außerdem auf dem Gebiet der Elektrotechnik einsetzbar, weil sie sehr gute elektrische Eigenschaften haben.

Weiterhin können die erfindungsgemäßen Formmassen beispielsweise zur Herstellung von folgenden Formkörpern bzw. Formteilen verwendet werden:
1. Innenausbauteile für Schienenfahrzeuge (FR)
2. Radkappen
3. Gehäuse von Kleintransformatoren enthaltenden Elektrogeräten
4. Gehäuse für Geräte zur Informationsverbreitung und -Übermittlung
5. Gehäuse und Verkleidung für medizinische Zwecke
6. Massagegeräte und Gehäuse dafür
7. Spielfahrzeuge für Kinder
8. Flächige Wandelemente
9. Gehäuse für Sicherheitseinrichtungen
10. Kfz-Spoiler
11. Wärmeisolierte Transportbehältnisse
12. Vorrichtung zur Haltung oder Versorgung von Kleintieren
13. Formteile für Sanitär- und Badeausrüstungen
14. Abdeckgitter für Lüfteröffnungen
15. Formteile für Garten- und Gerätehäuser
16. Gehäuse für Gartengeräte

Eine weitere Form der Verarbeitung ist die Herstellung von Formkörpern durch Tiefziehen aus vorher hergestellten Platten oder Folien.

Nachfolgend wird die Erfindung anhand von Beispielen sowie der lediglich ein bevorzugtes Ausführungsbeispiel darstellenden Zeichnung näher beschrieben. In der Zeichnung zeigen
- Fig. 1: eine bevorzugt ausgebildete Seiherschnecke als Abpressvorrichtung, schematisch im Längsschnitt,
- Fig. 2: schematisch einen konventionellen Mischkneter,
- Fig. 3: eine Abwicklung eines ersten Rotors eines für das Verfahren geeigneten Mischkneters und
- Fig. 4: eine Abwicklung des zweiten Rotors des oben genannten Mischkneters.

### Beispiele:

### Beispiel für die Komponente B):

Styrol/Acrylnitril-Copolymerisat mit einem Styrol/Acrylnitril-Verhältnis von 72 : 28 und einer Grenzviskosität von 0,55 dl/g (Messung in Dimethylformamid bei 20°C).

### Beispiele für die Komponente D):

D/50 (Einsatzprodukt für die nach dem erfindungsgemäßen Verfahren hergestellte Komponente D/18):

Pfropfpolymerisat von 40 Gew.-Teilen eines Copolymerisats aus Styrol und Acrylnitril im Verhältnis von 73 : 27 auf 60 Gew.-% teilchenförmigen vernetzten Polybutadienkautschuk (mittlerer Teilchendurchmesser d₅₀=0,3 µm), hergestellt durch Emulsionspolymerisation. Der wässrige Latex wird in der üblichen Weise mit Salzen bzw. mit Säuren gefällt, das Fällgut wird in fester, feuchter Form durch Filtration mit einer Wäsche isoliert. Der Wassergehalt beträgt 50 %.

### D/30 (Einsatzprodukt für die erfindungsgemäße Herstellung des Copolymerisats):

Pfropfpolymerisat von 40 Gew.-Teilen eines Copolymerisats aus Styrol und Acrylnitril im Verhältnis von 73 : 27 auf 60 Gew.-% teilchenförmigen vernetzten Polybutadienkautschuk (mittlerer Teilchendurchmesser d₅₀=0,3 µm), hergestellt durch Emulsionspolymerisation. Der wässrige Latex wird in der üblichen Weise mit Salzen bzw. mit Säuren gefällt, das Fällgut wird filtriert, mit Wasser gewaschen und zentrifugiert. Das Produkt hat eine Feuchte von 30 %.

### Erfindungsgemäßes Beispiel 1

### Herstellung von Pfropfpolymer D/18:

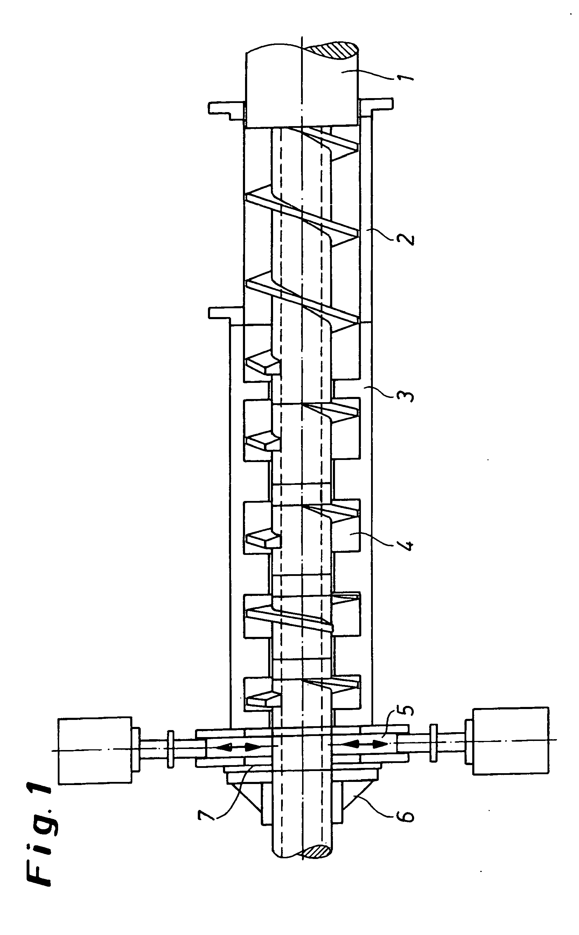

Das Pfropfpolymer D/50 wurde aus der Filtration als Feuchtgut mit einem Wassergehalt von 49 % entnommen und mit einer Rate von 534 kg/h einer bevorzugt verwendeten und in Fig. 1 dargestellten Seiherschnecke vom Typ Expeller® der Fa. Anderson International, Cleveland, Ohio zugeführt. Die Schnecke 1 wies einen Durchmesser von 6 inch auf. Einem Einzugsgehäuse 2 von 11 inch Länge folgte ein Entwässerungsgehäuse 3 von 22 inch Länge, das aus Stäben 4 aufgebaut war. Die Gangsteigung der Schnecke 1 betrug im Einzug 45 inch und komprimierte schrittweise auf 2 inch. Der Kerndurchmesser der Schnecke 1 betrug über die gesamte Länge der Schnecke 1 3,25 inch. Der Austrittsquerschnitt konnte mit Schiebern 5 teilweise verschlossen werden. Am Austritt aus der Schnecke 1 wurde ein eventuell vorhandener Grobanteil durch vier rotierende Messer 6 und feststehende Messer 7 zerkleinert.

Die Schnecke 1 wurde mit einer Drehzahl von 70 upm betrieben. Durch die Schlitze des Entwässerungsgehäuses 3 wurden 215 kg/h Wasser abgepresst. Der Feststoffanteil im Wasser war < 1 %. An der Düse erhielt man 319 kg/h an entwässertem Pfropfpolymer mit 15 % Restfeuchte. Das Polymer verließ die Maschine überwiegend in Pulverform. Durch eine Siebanalyse wurde festgestellt, dass der plastifizierte Anteil kleiner als 5 % war. Die Temperatur der Polymeren blieb während der Verarbeitung < 50°C.

### Herstellung von ABS Copolymer:

Das derart entwässerte Pfropfpolymer D/18 wurde mit 66,2 kg/h dem gegenläufigen Knetreaktor ORP12 aus Beispiel 1 von EP-A1-0 867 463 (siehe Vergleichsbeispiel 1) zugeführt. Außerdem wurden 18,75 kg/h des Thermoplasten B) als Schmelze mit einer Temperatur von 230°C zugegeben. Die Rotoren drehten wie im Vergleichsbeispiel 1 mit einer Drehzahl von 100 bzw. 25 Umdrehungen pro Minute. Die Apparatewände wurden mit einer Temperatur von 280°C beheizt. Die eingetragene mechanische Leistung betrug 7 kW. Das Produkt wurde mit 75 kg/h bei einer Temperatur von 225°C extrudiert. Die mechanische Leistung war gegenüber dem Vergleichsbeispiel 1 mehr als verdoppelt und die Durchsatzleistung um ca. 75 % gesteigert.

### Vergleichsbeispiel 1:

### Das Beispiel erfolgte analog Beispiel 1 von EP-A1-0 867 463.

Die eingetragene mechanische Leistung betrug 3 kW. Als der Durchsatz gesteigert wurde, traten Wasserdampfblasen im extrudierten Strang auf und ein Teil des Pulvers ging mit den Brüden verloren.

### Erfindungsgemäßes Beispiel 2:

54,27 kg/h des teilweise mittels einer Zentrifuge entwässerten Pfropfpolymeren D/30 mit 26,3 % Wasser und 20 kg/h der Thermoplastschmelze B) mit einer Temperatur von 230°C wurden dem gleichsinnigen Knetreaktor CRP12 zugeführt.

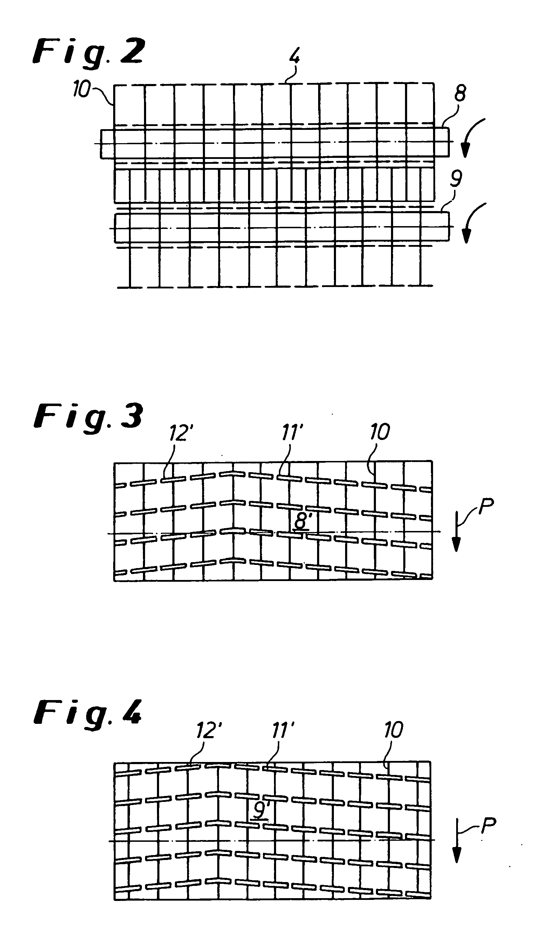

Der Knetreaktor hatte in der konventionellen und in Fig. 2 schematisch dargestellten Ausführung zwei gleichsinnig drehende Rotoren. Die Rotoren setzten sich jeweils aus einer beheizten Kernwelle 8 und 9 zusammen, welche jeweils eine Vielzahl paralleler Scheiben 10 trugen, wobei an einem Rotor vier und am anderen Rotor fünf Knetbarren 11 an jeder Scheibe 10 angebracht waren. Die Drehzahlen der Rotoren hatten das umgekehrte Verhältnis. Die Knetbarren hatten einen Anstellwinkel von 5° bzw. 4° zur Längsachse der Maschine, um die Transportwirkung der Rotoren zu verbessern. Insgesamt wies jeder Rotor elf Knetbarren tragende Scheiben 10 auf.

Schließlich zeigen die Fig. 3 und 4 die Anordnung der Knetbarren gemäß dem erfindungsgemäßen Ausführungsbeispiel. Dargestellt ist jeweils die Abwicklung der Rotoren 8' und 9'. Die Produktflussrichtung verläuft in der Darstellung von rechts nach links. Die Drehrichtung der Rotoren ist durch Pfeile P markiert. An der achten Scheibe in Produktflussrichtung erfolgt eine Richtungsumkehr der Anstellung der Knetbarren von vorwärtsfördernden Knetbarren 11' zu rückfördernden Knetbarren 12'. Die vorwärtsfördernden Knetbarren 11' weisen dabei in der ursprünglichen Ausführung einen Winkel von 4° bzw. 5° zur Längsachse des Mischkneters auf. Die rückfördernden Knetbarren 12' weisen beispielhaft einen Winkel von 8,75° bzw. 7° zur Längsachse des Mischkneters auf.

Die Rotoren drehten mit einer Drehzahl von 100 bzw. 80 Umdrehungen pro Minute. Die Gehäuse des Knetreaktors wurden mit Thermalöl von 200°C beheizt. Die Wellen wurden beim Anfahren der Maschine beheizt. Danach wurde die Zufuhr von Thermalöl zu den Wellen abgesperrt. Die Maschine brachte eine Knetleistung von 0,24 kWh/kg ein. Das Produkt wurde mit ca. 60 kg/h bei einer Temperatur von 210°C aus dem Mischkneter abgezogen. Der Betriebspunkt war langzeitstabil.

### Vergleichsbeispiel 2:

Der Knetreaktor wurde in seiner üblichen Ausführungsform eingesetzt, d.h. alle Knetbarren hatten eine förderaktive Anstellung von 5° bzw. 4°. 47,26 kg/h des teilweise entwässerten Pfropfpulvers D/30 und 16,66 kg/h der Thermoplastschmelze B) mit einer Temperatur von 230°C werden dem Kneter zugeführt. Die Rotoren drehten mit einer Drehzahl von 100 bzw. 80 Umdrehungen pro Minute. Die Gehäuse des Knetreaktors wurden mit Thermalöl von 200°C beheizt. Die Wellen wurden beim Anfahren der Maschine beheizt. Danach wurde die Zufuhr von Thermalöl zu den Wellen abgesperrt. Das Produkt wurde mit durchschnittlich 50 kg/h extrudiert; es ergab sich jedoch kein stabiler Betrieb. Nach ca. 30 Minuten Betriebszeit traten Gasblasen im Strang auf und das Pfropfpulver wurde mit dem Wasserdampf aus dem Kneter getragen, da die Plastifizierung unzureichend war.

## Patentansprüche

1. Verfahren zur Herstellung von elastomermodifizierten Thermoplasten durch Mischen von Elastomer D), welches durch Pfropfpolymerisation mit einer Hülle aus einem thermoplastischen Kunststoff A) umgeben ist, mit einem thermoplastischen Kunststoff B), **dadurch gekennzeichnet, dass** das feuchte gepfropfte Elastomer D) auf 1 bis 50 Gew.-% Restfeuchte vorentwässert und in den als Schmelze vorliegenden thermoplastischen Kunststoff B) eingemischt wird, wobei das Schmelzen des Elastomeren D), das Legieren des Elastomeren D) mit der Schmelze des thermoplastischen Kunststoffs B), sowie die Entfernung von weiteren organischen flüchtigen Bestandteilen C) in einem kontinuierlich durchlaufenen Knetreaktor mit Knetbarren erfolgt, der so aufgebaut ist, dass ein Teil der Knetbarren das Kunststoffmaterial in Richtung des Reaktoreinlasses rückfördert.

2. Verfahren nach Anspruch 1, **dadurch gekennzeichnet, dass** das Vorentwässern des Elastomeren D) in einer Zentrifuge, einer Entwässerungsschnecke oder in einer Sequenz beider Aggregate erfolgt.

3. Verfahren nach Anspruch 2, **dadurch gekennzeichnet, dass** das Vorentwässern des Elastomeren D) in einer Zentrifuge und einer nachgeschalteten Entwässerungsschnecke erfolgt, wobei das Elastomer D) nach der Vorentwässerung eine Restfeuchte von 10 bis 23 Gew.-% aufweist.

4. Verfahren nach einem der Anspruche 1 bis 3, **dadurch gekennzeichnet, dass** die zum Aufschmelzen, Aufheizen und Ausdampfen der Polymermischung notwendige Energie mechanisch über die Knetwirkung der Rotoren und thermisch über die Gehäuseflächen des Knetreaktors eingebracht wird, wobei das Verhältnis zwischen in die Mischung einzubringender mechanischer und thermischer Energie von 4 : 1 bis 1: 6 beträgt.

5. Verfahren nach einem der Ansprüche 1 bis 4, **dadurch gekennzeichnet, dass** der Kautschuk für das gepfropfte Elastomer D) ausgewählt ist aus der Reihe: Butadienkautschuk, Acrylnitril Butadienkautschuk, Styrol- Acrylnitril-Butadienkautschuk.

6. Verfahren nach einem der Ansprüche 1 bis 5, **dadurch gekennzeichnet, dass** die Hülle aus Kunststoff A) ausgewählt ist aus der Reihe Styrol-Acrylnitril-(SAN)-Copolymere, Polystyrol, Polymethylmethacrylat, Polyvinylchlorid, Polycarbonat, Polybutylenterephthalat, Polyoxymethylen, Polymethylmethacrylat, Polyphenylensulfid, Polysulfone, Polyethersulfone und Polyamide und Mischungen dieser Thermoplasten.

7. Verfahren nach einem der Ansprüche 1 bis 6, **dadurch gekennzeichnet, dass** der Thermoplast B) ausgewählt ist aus der Reihe Styrol-Acrylnitril-(SAN)-Copolymere, Polystyrol, Polymethylmethacrylat, Polyvinylchlorid, Polycarbonat, Polybutylenterephthalat, Polyoxymethylen, Polymethylmethacrylat, Polyphenylensulfid, Polysulfone, Polyethersulfone und Polyamide und Mischungen dieser Thermoplasten.

8. Verfahren nach einem der Ansprüche 1 bis 7, **dadurch gekennzeichnet, dass** die Rückförderung der Knetbarren im Auslassbereich des Knetreaktors erfolgt.

9. Knetreaktor, zur Durchführung eines Verfahrens nach einem der Ansprüche 1 bis 8, mindestens ausgestattet mit zwei achsparallelen Wellen (8', 9'), wobei sich auf beiden Wellen (8', 9') Scheiben (10) mit an ihrem Umfang angeordneten Knetbarren (11'; 12') zur Förderung des Reaktionsgemisches befinden, mit einem die Einbauten umschließenden Gehäuse mit mindestens zwei Einläsen und einem Austrag für das Produkt, einem Antriebsmittel für die gleich- oder gegensinnige Rotation der Wellen (8', 9') sowie einer Heiz-/Kühleinrichtung, **dadurch gekennzeichnet, dass** ein Teil der Knetbarren (11', 12') einen solchen Anstellwinkel zu ihrer Bewegungsrichtung aufweisen, dass sie bei einer durch das Antriebsmittel bewirkten Drehung der Wellen (8', 9') das Produkt vom Austrag weg in Richtung der Einlässe fördern.

10. Knetreaktor nach Anspruch 9, **dadurch gekennzeichnet, dass** die an den drei bis fünf zum Austrag nächststehend angeordneten Scheiben (10) befindlichen Knetbarren (12') einen Anstellwinkel mit Rückforderungswirkung aufweisen.

11. Formmassen hergestellt aus elastomermodifizierten Thermoplasten erhalten nach einem Verfahren nach einem der Ansprüche 1 bis 8.

12. Formkörper hergestellt aus Formmassen gemäß Anspruch 11.

## Claims

1. Process for the production of elastomer-modified thermoplastics by mixing of elastomer D), which, as a result of graft polymerization, is surrounded by an envelope of a thermoplastic A), with a thermoplastic B), **characterized in that** the moist grafted elastomer D) is pre-dewatered to a residual moisture content of 1 to 50 wt.% and mixed into the molten thermoplastic B), the melting of the elastomer D), the alloying of the elastomer D) with the melt of the thermoplastic B) and also the removal of further organic volatiles C) being effected in a continuous kneaded reactor which is equipped with kneading bars and is constructed such that some of the kneading bars convey the plastics material back in the direction of the reactor inlet.

2. Process according to Claim 1, **characterized in that** the pre-dewatering of the elastomer D) is effected in a centrifuge, a dewatering screw or in a sequence of the two assemblies.

3. Process according to Claim 2, **characterized in that** the pre-dewatering of the elastomer D) is effected in a centrifuge and a downstream dewatering screw, the elastomer D) having a residual moisture content of 10 to 23 wt.% after the pre-dewatering.

4. Process according to any one of Claims 1 to 3, **characterized in that** the energy necessary for melting, heating and devolatilizing the polymer mixture is introduced mechanically via the kneading action of the rotors and thermally via the housing surfaces of the kneaded reactor, the ratio between mechanical and thermal energy to be introduced into the mixture being in the range from 4:1 to 1:6.

5. Process according to any one of Claims 1 to 4, **characterized in that** the rubber for the grafted elastomer D) is selected from the group consisting of butadiene rubber, acrylonitrile-butadiene rubber and styrene-acrylonitrile-butadiene rubber.

6. Process according to any one of Claims 1 to 5, **characterized in that** the envelope of plastic A) is selected from the group consisting of styreneacrylonitrile (SAN) copolymers, polystyrene, polymethyl methacrylate, polyvinyl chloride, polycarbonate, polybutylene terephthalate, polyoxymethylene, polymethyl methacrylate, polyphenylene sulphide, polysulphones, polyether sulphones and polyamides and mixtures of these thermoplastics.

7. Process according to any one of Claims 1 to 6, **characterized in that** the thermoplastic B) is selected from the group consisting of styreneacrylonitrile (SAN) copolymers, polystyrene, polymethyl methacrylate, polyvinyl chloride, polycarbonate, polybutylene terephthalate, polyoxymethylene, polymethyl methacrylate, polyphenylene sulphide, polysulphones, polyether sulphones and polyamides and mixtures of these thermoplastics.

8. Process according to any one of Claims 1 to 7, **characterized in that** the back-conveying of the kneading bars is effected in the outlet region of the kneaded reactor.

9. Kneaded reactor for carrying out a process according to any one of Claims 1 to 8, at least equipped with two axially parallel shafts (8', 9') which are each equipped with discs (10) having peripherally disposed kneading bars (11', 12') for conveying the reaction mixture, with a housing which encloses the internals and is equipped with at least two inlets and an exit for the product, a drive means for the co- or contra-rotation of the shafts (8', 9') and also a heating/cooling appliance, **characterized in that** some of the kneading bars (11', 12') have such a pitch angle relative to their direction of movement that when the shafts (8', 9') turn in response to the action of the drive means they convey the product away from the exit and in the direction of the inlets.

10. Kneaded reactor according to Claim 9, **characterized in that** the kneading bars (12') situated on the three to five discs (10) nearest the exit have a pitch angle with a back-conveying effect.

11. Moulding compositions produced from elastomer-modified thermoplastics obtained according to a process according to any one of Claims 1 to 8.

12. Moulded articles produced from moulding compositions according to Claim 11.

## Revendications

1. Procédé pour la préparation de thermoplastiques modifiés par des élastomères par mélange d'un élastomère D), qui est entouré par polymérisation greffée d'une enveloppe d'un matériau synthétique A) thermoplastique, avec un matériau synthétique B) thermoplastique, **caractérisé en ce que** l'élastomère D) humide greffé est déshydraté au préalable à 1 jusqu'à 50% en poids d'humidité résiduelle et est incorporé par mélange dans le matériau synthétique B) thermoplastique se trouvant sous forme de masse fondue, la fusion de l'élastomère D), le compoundage de l'élastomère D) avec la masse fondue du matériau synthétique B) thermoplastique ainsi que l'élimination des autres constituants C) volatils organiques étant réalisés dans un réacteur à malaxage traversé en continu avec des barres de malaxage, qui est construit de telle manière qu'une partie des barres de malaxage transporte en retour le matériau synthétique vers l'entrée du réacteur.

2. Procédé selon la revendication 1, **caractérisé en ce que** la déshydratation préalable de l'élastomère D) est réalisée dans une centrifugeuse, une vis sans fin de déshydratation ou dans une séquence de ces deux appareils.

3. Procédé selon la revendication 2, **caractérisé en ce que** la déshydratation préalable de l'élastomère D) est réalisée dans une centrifugeuse et une vis sans fin de déshydratation disposée en aval, l'élastomère D) présentant une humidité résiduelle de 10 à 23% en poids après la déshydratation préalable.

4. Procédé selon l'une quelconque des revendications 1 à 3, **caractérisé en ce que** l'énergie nécessaire pour la fusion, le chauffage et l'évaporation du mélange polymère est introduite mécaniquement via l'effet de malaxage des rotors et thermiquement via les surfaces du bâti du réacteur à malaxage, le rapport entre l'énergie mécanique et l'énergie thermique à introduire étant de 4:1 à 1:6.

5. Procédé selon l'une quelconque des revendications 1 à 4, **caractérisé en ce que** le caoutchouc pour l'élastomère D) greffé est choisi dans la série : caoutchouc de butadiène, caoutchouc d'acrylonitrile-butadiène, caoutchouc de styrène-acrylonitrilé-butadiène.

6. Procédé selon l'une quelconque des revendications 1 à 5, **caractérisé en ce que** l'enveloppe en matériau synthétique A) est choisie dans la série formée par les copolymères de styrène-acrylonitrile-(SAN), le polystyrène, le poly(méthacrylate de méthyle), le poly(chlorure de vinyle), le polycarbonate, le poly(téréphtalate de butylène), le polyoxyméthylène, le poly(méthacrylate de méthyle), le poly(sulfure de phénylène), les polysulfones, les polyéthersulfones et les polyamides et les mélanges de ces thermoplastiques.

7. Procédé selon l'une quelconque des revendications 1 à 6, **caractérisé en ce que** le thermoplastique B) est choisi dans la série formée par les copolymères de styrène-acrylonitrile-(SAN), le polystyrène, le poly(méthacrylate de méthyle), le poly(chlorure de vinyle), le polycarbonate, le poly(téréphtalate de butylène), le polyoxyméthylène, le poly (méthacrylate de méthyle), le poly(sulfure de phénylène), les polysulfones, les polyéthersulfones et les polyamides et les mélanges de ces thermoplastiques.

8. Procédé selon l'une quelconque des revendications 1 à 7, **caractérisé en ce que** le transport en retour des barres de malaxage est réalisé dans la zone de sortie du réacteur à malaxage.

9. Réacteur à malaxage destiné à la réalisation d'un procédé selon l'une quelconque des revendications 1 à 8, au moins équipé de deux arbres à axes parallèles (8', 9'), des disques (10) se trouvant sur les deux arbres (8', 9'), comportant des barres de malaxage (11', 12') disposées en leur périphérie pour le transport du mélange réactionnel avec un châssis entourant les pièces, présentant au moins deux entrées et une sortie pour le produit, un moyen d'entraînement pour la rotation dans le même sens ou un sens opposé l'un à l'autre des arbres (8', 9') ainsi qu'un dispositif de chauffage/refroidissement, **caractérisé en ce qu'**une partie des barres de malaxage (11', 12') présente un angle de réglage par rapport à leur sens de mouvement tel qu'elles transportent le produit en l'éloignant de la sortie vers les entrées lors d'une rotation des arbres (8', 9') provoquée par le moyen d'entraînement.

10. Réacteur à malaxage selon la revendication 9, **caractérisé en ce que** les barres de malaxage (12') se trouvant sur les trois à cinq disques (10) les plus proches de la sortie présentent un angle de réglage avec un effet de transport en retour.

11. Masses de moulage, réalisées à partir de thermoplastiques modifiés par des élastomères obtenus selon un procédé selon l'une quelconque des revendications 1 à 8.

12. Corps moulé réalisé à partir des masses de moulage selon la revendication 11.
